# EUROPEAN PATENT SPECIFICATION

(11) **EP 2 480 651 B1**
(45) Date of publication and mention of the grant of the patent: **03.08.2016**
(21) Application number: 10747052.8
(22) Date of filing: 30.08.2010
(51) Int. Cl.: C11D 3/48, C11D 3/12, A01N 25/26, C01B 33/44, C09C 1/42, A01N 43/40, A61K 8/26, A61Q 17/00, A61K 8/02, A61Q 5/00, A61Q 19/10

(54) **AN ANTIMICROBIAL PARTICLE AND A PROCESS FOR PREPARING THE SAME**
ANTIMIKROBIELLES TEILCHEN UND DESSEN HERSTELLUNGSVERFAHREN
PARTICULE ANTIMICROBIENNE ET SONT PROCÉDÉ DE FABRICATION

(30) Priority: 24.09.2009 IN MU22222009; 10.11.2009 EP 09175488
(43) Date of publication of application: 01.08.2012
(73) Proprietor: Unilever N.V., 3013 AL Rotterdam (NL); Unilever PLC, London Greater London EC4Y 0DY (GB)
(72) Inventor: BHATTACHARYA, Arpita, Bangalore 560 066 (IN); GHOSH DASTIDAR, Sudipta, Bangalore 560 066 (IN); IYER, Vidula, Bangalore 560 066 (IN); JAYARAMAN, Suresh, Sambamurthy, Bangalore 560 066 (IN); SAJI, Maya, Treesa, Bangalore 560 066 (IN)
(74) Representative: van den Brom, Coenraad Richard
(86) International application number: PCT/EP2010/062618
(87) International publication number: WO 2011/036031

(56) References cited:
- EP-A1- 0 319 168
- EP-A2- 0 265 101
- WO-A1-99/05249
- WO-A1-2009/118421
- US-A- 4 916 095
- US-A- 5 137 568
- US-A- 6 165 485

## Description

### Field of the Invention

This invention relates to a bipolar antimicrobial particle for use in laundry detergent compositions, fabric conditioners, personal care and cosmetic compositions and a process for making the same.

### Background of the invention

Antimicrobials (or antimicrobial agents or biocides) are used widely in many technological fields like detergents and personal care, especially in laundry (including e.g. wash and fabric conditioning) and in personal care (including e.g. personal wash, shampoo and deodorant) compositions for giving antimicrobial activity to the substrate.

In today's world most people are concerned about their health, hygiene and appearance. Therefore most of the consumer products use antimicrobial as one of the key ingredients. People use antimicrobial in oral hygiene products, in skin lotions and creams, in anti-perspirant and in many other formats. The objective of this product format is generally to deliver a suitable antimicrobial agent to a target substrate.

Antimicrobial agents essentially reduce the microbial activity or inhibit growth of microorganisms on a surface or in a composition. In case of deodorants antimicrobial helps to reduce the malodour caused by the microbial activity in human sweat.

Likewise in case of laundry compositions adding an antimicrobial molecule in the formulation and depositing it through main-wash or after-wash will reduce the microbial activity in the washing liquor and on the fabric thus avoiding malodour on the fabric. To accommodate those consumer preferences, adding antimicrobial agents into detergent compositions has been proposed in the field to provide antimicrobial activity to detergent powders and to provide a benefit to fabric articles washed with such powders.

There are detergent powders available in the market comprising various antimicrobial agents.

The drawback of the currently available antimicrobial agents is that high amounts of antimicrobial get lost during the rinsing stages of fabric washing processes and retention of the antimicrobial agent in personal wash, personal care and oral hygiene remains to be desired. Therefore washed fabrics end up with a relatively low amount of adsorbed antimicrobial and high dosing and/or repeated use are required in personal wash, personal care and oral hygiene. Accordingly the dosage of antimicrobial in the respective washing products is generally higher than required to compensate for the loss. Since antimicrobial agents are relatively expensive ingredients, it is desired to reduce the loss on rinsing.

Clay with adsorbed antimicrobial is also known in literature. After the absorption people add this organoclay to a polymer matrix to form a nanocomposite is also known.

WO2008/152417 (Fengge et al.) discloses a method of preparing a polymer nanocomposite having antimicrobial properties, comprising (i) contacting a polymeric antimicrobial agent with a clay to form an organoclay; and (ii) subsequently dispersing the organoclay in a polymeric matrix. This is believed to reduce the leaching of the polymeric antimicrobial agent from the composite. WO2008/152417 uses a material in which antimicrobial agents are entrapped and adsorbed onto the clay surface dispersed in a polymeric matrix and doesn't provide a robust solution of making an antimicrobial particle as a single entity with absolute increase in retention property.

Hence a particle with immobilized antimicrobial agent remains to be desired.

Our co-pending application WO 2009/118421 discloses a particle with bipolar toposelective characteristics, but it does not teach to provide a stabile antimicrobial agent immobilised on a carrier particle.

US 5,317,568 discloses the improvement of dispersibility of extender pigments in ink formulations by using as the extender pigment, a quaternary ammonium treated kaolin clay. However, the method disclosed in US 5,317,568 is not suitable for the purpose of the present invention

Another problem of known antimicrobial agents is their stability on fabric. Even if freshly treated fabric shows significant activity, the activity reduces significantly on storage.

At present the art does not provide an antimicrobial particle with improved retention on the fabric so that a higher amount of antimicrobial will stick to the fabric even after rinsing. This remains to be desired.

Another problem associated with currently available antimicrobial agents as used in cosmetic compositions and personal care compositions as well as personal wash and shampoo compositions is that improved stability remains to be desired as there are usually many other ingredients with which they may interact thereby reducing their stability.

### Objects

In view of the foregoing, it is an object of the present invention to provide a stable antimicrobial agent immobilised on a carrier particle.

It is a further objective to provide an antimicrobial particle with improved retention to the fabric so that larger amounts of antimicrobial will be available even after rinsing.

It is a further object of the invention to provide improved antimicrobial activity to fabrics and textiles.

It is yet a further object of the invention to provide lasting antimicrobial activity in personal care compositions.

It is yet a further object of the invention to provide improved antimicrobial action on skin.

It is still a further object of the invention to provide better hygiene when formulated in hand-wash compositions.

It is still a further object of the invention to provide better dental hygiene when formulated in an oral care and/or mouth wash compositions.

It is still a further object of the invention to provide better hygiene when formulated in shampoo compositions.

Surprisingly it has been found that antimicrobial molecules tagged by surface reaction onto naturally occurring asymmetric clay surfaces, act as an antimicrobial particle with improved retention properties with improved stability.

### Summary of the invention

Accordingly the present invention provides a bipolar antimicrobial particle,
a which precursor is an asymmetric 1:1 or 2:1:1 clay particle comprising alternating tetrahedral and octahedral sheets terminating with a tetrahedral sheet at one external surface plane and an octahedral sheet at another external surface plane,
b with an antimicrobial group attached to the coordinating cation on one of the said external surface plane selected from a quaternary ammonium material comprising a single alkyl or alkenyl long chain having an average chain length greater than or equal to C₂₀, or a quaternary ammonium material selected from Cetylpyridinium chloride (CPC), Cetyltrimethylammonium Chloride (CTAC), Cetyltrimethylammonium Bromide (CTAB), Benzalkonium Chloride (BKC), Benzethonium chloride, cetrimide, Quaternium, polyhexamethylene BH, antimicrobial alcohols as defined in claim 1, antimicrobial phenols as defined in claim 1, antimicrobial organic acids/salts as defined in claim 1, Zinc pyrithione, Ketoconazole, Octopirox ^{R} or combinations thereof, wherein said antibacterial group is attached to coordinating cations on the external surface of the octahedral surface plane.

In another aspect, the invention provides a detergent composition comprising antimicrobial particle of the invention. In another aspect the invention provides the use of the particles according to the invention for increasing antimicrobial activity on fabrics and textiles, preferably non-therapeutical.

In another aspect the invention provides the use of the particles according to the invention for increasing antimicrobial activity on skin and scalp, preferably non-therapeutical.

In another aspect the invention provides A process for preparing bipolar antimicrobial particle as defined in claims 1-2, comprising the steps of (a) contacting the precursor with a mineral acid (b) adjusting the pH of the solution above 8 (c) adding a antimicrobial molecule to the mixture (d) heating the mixture to a temperature of 50 - 150 °C for 30 minutes to 10 hours while stirring, and (e) separating the solid product comprising bipolar particulate antimicrobial.

In another aspect the invention provides a personal wash composition comprising antimicrobial particle of the invention and an acceptable base.

In another aspect the invention provides a deodorant composition comprising antimicrobial particle of the invention and an acceptable base.

These and other aspects, features and advantages will become apparent to those of ordinary skill in the art from a reading of the following detailed description. For the avoidance of doubt, any feature of one aspect of the present invention may be utilised in any other aspect of the invention. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

### Detailed description of the invention

### Precursor

The precursor of the particle with bipolar topospecific characteristics according to the present invention is preferably an asymmetric 1:1 or 2:1:1 clay particle having alternating tetrahedral and an octahedral sheets terminating with a tetrahedral and an octahedral sheet at exterior surface planes. Particle of 1:1 clay is particularly preferred as precursor.

According to the present invention preferred 1:1 clays include kaolinite and serpentine subgroups of minerals. The species included within the kaolinite subgroup include but are not limited to kaolinite, dickite, halloysite and nacrite.

The species within the serpentine subgroup include but are not limited to chrysolite, lizardite, and amesite.

According to the present invention preferred 2:1:1 clays include chlorite group of minerals. Chlorite is sometimes wrongly referred to as 2:2 clay by some mineralogists. The chlorite comprises tetrahedral-octahedral-tetrahedral sheets like 2:1 clays, with an extra weakly bound brucite like layer between tetrahedral layers.

The tetrahedral sheet preferably comprises coordinating tetrahedral cations of silicon. The tetrahedral sheet may also comprise isomorphously substituted coordinating tetrahedral cations which are not silicon. Isomorphously substituted coordinating tetrahedral cations include, but are not limited to, cations of aluminum, iron or boron.

The octahedral sheet preferably comprises coordinating octahedral cation of aluminum. The octahedral sheet may also comprise isomorphously substituted coordinating octahedral cations which are not aluminium. Isomorphously substituted coordinating octahedral cations include cations of magnesium or iron.

The antimicrobial agent is attached to the coordinating cations on the exterior side of one of the external surface planes. Accordingly, the antimicrobial molecule is attached to coordinating cations on the exterior side of the tetrahedral sheet. Alternatively, the antimicrobial molecule is attached to the coordinating cations on the exterior side of the octahedral sheet. According to a further aspect, coordinating cations on the exterior side of each of the tetrahedral and the octahedral surface sheets are attached to a antimicrobial molecule, with the proviso that the antimicrobial molecule attached to the coordinating cations on the exterior side of the tetrahedral surface sheet is not identical to the molecule attached to the coordinating cations on the exterior side of the octahedral surface sheet.

The antimicrobial molecule is preferably attached to the coordinating cations on the external surface of the octahedral surface plane and is not preferably attached to coordination cations of non-exterior tetrahedral or octahedral plane or on the interior side of the surface sheets.

In the above mentioned particulate antimicrobial the clay: antimicrobial ratio is between 1:0.001 and 1:0.1, more preferably between 1:0.01 and 1:0.05, most preferably about 1:0.018.

### Process

Any chemical reaction or series of reactions wherein an antimicrobial molecule is attached selectively to coordinating cations on the exterior plane of either the tetrahedral or the octahedral surface plane of asymmetric clay can be used to prepare the bipolar particulate antimicrobial according to the present invention. In order to obtain a true bipolar antimicrobial particle it is preferred that the reaction is selective to only one of the exterior planes. By selective is meant that more than 50% of the total antimicrobial molecule is present on one of the exterior planes, preferably more than 75%, more preferably than 80%, still more preferably than 90%, even more preferably than 95%, or even more than 99%. The chemical reaction or series of reactions wherein the same antimicrobial molecule attached to coordinating cations of both the surface sheets, viz octahedral and tetrahedral, are therefore not preferred.

The particle with bipolar characteristics may have two distinct regions on its surface having non-identical surface characteristics. It is particularly preferred that the particle has two spatially distinct exterior faces having distinct surface characteristics. It is envisaged that by selecting specific antimicrobial molecule having specific group, and selectively attaching them to coordinating cations of tetrahedral and/or octahedral surface sheets, it is possible to impart anisotropic characteristics of various types to the surface of particle with bipolar characteristics.

According to another aspect, the invention provides a process for preparing a bipolar antimicrobial particle which precursor is an asymmetric 1:1 or 2:1:1 clay particle having alternating tetrahedral and octahedral sheets terminating with a tetrahedral sheet at one external surface plane and an octahedral sheet at another external surface plane, comprising the steps of contacting the precursor with a mineral acid, adding a antimicrobial molecule to the mixture, adjusting the pH of the solution above 8, heating the mixture to a temperature of 50 - 150 °C for about 30 minutes to 10 hours while stirring, and separating the solid product comprising bipolar particulate antimicrobial. When temperature is greater than 100°C is applied, a pressure vessel is preferred.

### Treating the precursor clay with a mineral acid

At first the raw clay is treated with a mineral acid preferably hydrochloric acid. The hydrochloric acid is used in a concentration range of 0.01(N) to 1(N), preferably about 0.1(N). The clay particle with the acid is then stirred. The stirring is typically done for 10-60 minutes, preferably about 30 minutes.

### Adjusting the pH

After treating the precursor with mineral acid for about 30 minutes, pH of the system is adjusted to above 8 by adding e.g. 0.1 (M) NaOH to the solution.

### Adding a desired antimicrobial molecule

After treating the clay with hydrochloric acid it is preferred that the desired antimicrobial molecules were added to the dispersion. The antimicrobial molecules are then added in a concentration of 0.001 to 30 percent of the total weight of the dispersion, preferably 0.01 to 5%.

### Heating the solution while stirring

After adding the antimicrobial molecule and adjusting the pH of the dispersion, the solution is heated preferably for between 1 to 10 hours, preferably 4 to 8 hrs and more preferably about 6 hrs while stirring at 50°C to 150°C preferably 70°C to 90°C more preferably at 80°C while stirring.

### Separating the solid product comprising the bipolar particulate antimicrobial.

After the reaction, the dispersion mixture is preferably centrifuged to obtain the bipolar antimicrobial particle as residue. Then it is preferably washed with copious amount of water and subsequently with a ketone solvent (e.g. acetone). After that it is dried in an oven to get the final product.

In this reaction, antimicrobial molecule is attached to the coordinating cations of the octahedral sheet preferable by covalent bonding. The antimicrobial particle made by this process has different wettability characteristics for two external surface planes.

### Antimicrobial group

The anti micriobial group is selected from the group of Quaternary ammonium salts such as Cetylpyridinium chloride (CPC), Cetyltrimethylammonium Chloride (CTAC), Cetyltrimethylammonium Bromide (CTAB), Benzalkonium Chloride (BKC), Benzethonium chloride, cetrimide, Quaternium, polyhexamethylene BH etc or from the group of antimicrobial alcohols which are Phenoxy ethanol, benzyl alcohol, dichlorobenzyl alcohol, dimethyl oxazolidine, DMDM Hydantoin, 2-bromo-2-nitropropane-1,3-diol, diazolidinyl urea, hexachlorophene or from a group of antimicrobial phenols which are Triclosan, Thymol, dichlorophenol, 2-chloro-4-fluoro phenol, tetrafluorobenzoic acid, cresol, hexylresorcinol, microlides or from the group of antimicrobial organic acids/salts which are Benzoic acid/ sodium benzoate, Salicylic Acid/ Sodium Salicylate, Sorbic Acid/ Potassium Sorbate, sodium hydroxymethyl glycinate, cyclohexane diacetic acid monoamide, chloronicotinic acids, succinic acid, Peracetic acid or Zinc pyrithione, Ketoconazole, Octopirox ^{R}, or combinations thereof.

These antimicrobials are selected for the purpose of present invention to provide protection against skin bacteria such as *Propionibacteria spp., Corynebacteria spp., Actinobacteriales, Staphylococci spp.* (e.g. *S. epidermidis*), *Lactobacilales, Clostridiales, β-proteobacteria, γ-proteobacteria, α-proteobacteria, Flavobacteriales, Bacteriodales, Malassezia* yeasts *(e.g. Malassezia furfur* and *Malassezia globosa) etc.*

### Antimicrobial particles in detergent composition

The present invention provides a detergent composition for improved antimicrobial action on the fabrics and textiles. The particulate antimicrobial is preferably delivered to the fabric by a detergent composition. This detergent composition may be made by any conventional process.

The particulate antimicrobial is preferably incorporated in 0.01% to 10% by weight of the detergent composition, more preferably from 0.1% to 5% by weight of the composition.

Different kinds of surfactants may also be included in the detergent composition. Anionic, cationic, nonionic or zwitterionic surfactant or combinations thereof may be used in the detergent composition. In general, the surfactants of the surfactant system may be chosen from the surfactants described well known textbooks like "Surface Active Agents" Vol. 1, by Schwartz & Perry, Interscience 1949, Vol. 2 by Schwartz, Perry & Berch, Interscience 1958, and/or the current edition of "McCutcheon's Emulsifiers and Detergents" published by Manufacturing Confectioners Company or in "Tenside-Taschenbuch", H. Stache, 2nd Edn., Carl Hauser Verlag, 1981.

The surfactant is preferably incorporated in 5% to 50% by weight of the detergent composition, preferably at least 10% or even more than 15%, while generally less than 40% and even less than 30%. Although any concentration of surfactant may be used, suitable concentration is in the range of 0.5 to 3 grams per liter of the water after dissolution of the detergent composition into 10-60 liters of water for washing.

Apart from that, builders may also be included in the detergent composition. Preferred builders include alkali metal carbonates, borates, bicarbonates, silicates, sulphates and chlorides. Specific examples of such salts include sodium and potassium tetraborates, perborates, bicarbonates, carbonates, and sulphates. Phosphate builder (ex. STPP) may also be included. The builder is preferably incorporated in 10% to 50% by weight of the detergent composition.

Preferably 0% to 10% minors may also be incorporated in the detergent composition. These minors include perfumes, colours, pH modifier etc.

The detergent composition is suitable for any kind of laundry and machine-wash (with horizontal axis or vertical axis) applications and for any kind of fabric like cotton, polyester, polycotton etc.

### Antimicrobial particles in fabric conditioner

According to another aspect of the present invention provides a fabric after-wash composition. The antimicrobial particle is preferably delivered to the fabrics and textiles through a fabric conditioner composition. This fabric conditioner is made by usual way of making any fabric conditioner composition.

The particulate antimicrobial is preferably incorporated in 0.01% to 10% by weight of the detergent composition, more preferably from 0.1% to 5% by weight of the composition.

The wash component will preferably include a fabric softening and/or conditioning compound (hereinafter referred to as "fabric softening compound"), which may be a cationic or nonionic compound, as commonly used in the art.

The fabric softening compounds may be water insoluble quaternary ammonium compounds. The compounds may be present in amounts of up to 8% by weight (based on the total amount of the composition) in which case the compositions are considered dilute, or at levels from 8% to about 50% by weight, in which case the compositions are considered concentrates.

Compositions suitable for delivery during the rinse cycle may also be delivered to the fabric in the tumble dryer if used in a suitable form.

Suitable cationic fabric softening compounds are substantially water-insoluble quaternary ammonium materials comprising a single alkyl or alkenyl long chain having an average chain length greater than or equal to C₂₀ or, more preferably, compounds comprising a polar head group and two alkyl or alkenyl chains having an average chain length greater than or equal to C₁₄. Preferably the fabric softening compounds have two long chain alkyl or alkenyl chains each having an average chain length greater than or equal to C₁₆. Most preferably at least 50% of the long chain alkyl or alkenyl groups have a chain length of C₁₈ or above.

Substantially water-insoluble fabric softening compounds are defined as fabric softening compounds having a solubility of less than 1 x 10-3 wt % in demineralised water at 20°C. Preferably the fabric softening compounds have a solubility of less than 1 x 10-4 wt%, more preferably less than 1 x 10-8 to 1 x 10-6 wt%.

The compositions may alternatively or additionally contain water-soluble cationic fabric softeners. The compositions may comprise a cationic fabric softening compound and oil. The compositions may alternatively or additionally contain the polyol polyester (eg, sucrose polyester) compounds. The compositions may alternatively or additionally contain nonionic fabric softening agents such as lanolin and derivatives thereof. The compositions may also suitably contain a nonionic stabilising agent. Suitable nonionic stabilising agents are linear C₈ to C₂₂ alcohols alkoxylated with 10 to 20 moles of alkylene oxide, C₁₀ to C₂₀ alcohols, or mixtures thereof.

The composition can also contain fatty acids, for example C₈ to C₂₄ alkyl or alkenyl monocarboxylic acids or polymers thereof. The fabric conditioning compositions may include soil release polymers such as block copolymers of polyethylene oxide and terephthalate; amphoteric surfactants; zwitterionic quaternary ammonium compounds; and nonionic surfactants.

The fabric conditioning compositions may be in the form of emulsions or emulsion precursors thereof.

Other optional ingredients include emulsifiers, electrolytes (for example, sodium chloride or calcium chloride) preferably in the range from 0.01 to 5% by weight, pH buffering agents, and perfumes (preferably from 0.1 to 5% by weight).

Minors like perfume carriers, fluorescers, colourants, hydrotropes, antifoaming agents, antiredeposition agents, enzymes, opacifiers, dye transfer inhibitors, anti-shrinking agents, anti-spotting agents etc. can also be added to the formulation.

### Antimicrobial particles in shampoo composition

This shampoo composition is made by usual way of making any shampoo composition.

The particulate antimicrobial is preferably incorporated in 0.01% to 10% by weight of the shampoo composition, more preferably from 0.1% to 5% by weight of the composition.

The shampoo composition may comprise of an anionic surfactant selected from alkyl ether sulphate, alkyl sulphate or combinations thereof. Commonly used anionic surfactants are C₁₀ to C₁₈ alkyl sulphate, and C₁₀ to C₁₈ alkyl ether sulphates containing 1 to 5 moles of ethylene oxide. Apart from the above mentioned surfactant a number of other types of anionic surfactant may also be included.

The shampoo composition may also comprise of a sunscreen, or a mixture thereof, for photo protection.

The shampoo composition may additionally comprise of co-surfactants such as C₁₀-C₁₈ alkyl or alkylamido propyl betaine, C₁₀-C₁₈ fatty acid alkanolamide or mixtures thereof.

The shampoo composition of the invention may also include minors which are commonly employed in shampoos like foam boosters, viscosity-adjusting agents, pearlescers, perfumes, dyes, colouring agents, thickeners, conditioning agents, proteins, polymers, buffering agents, preservatives etc.

For antidandruff action on hair and scalp it is preferable that the antimicrobial agent, for the reaction with clay, to be used in shampoo composition may selected from Salicylic acid, Zinc pyrithione, Ketoconazole, Octopirox ^{R} etc to protect against the dandruff causing yeasts.

### Antimicrobial for cosmetic application

The present invention provides a cosmetic composition for improved antimicrobial actions on skin. The particulate antimicrobial is preferably delivered to the skin through a cosmetic composition. This cosmetic composition is made by usual way of making any skin formulation.

The particulate antimicrobial is preferably incorporated in 0.05% to 10% by weight of the skin composition, more preferably from 0.1% to 10%, most preferably from 0.2 % to 5 % by weight of the composition.

The skin cosmetic composition preferably comprises a cosmetically acceptable vehicle to act as a diluent, dispersant or carrier for other materials present in the composition, so as to facilitate their distribution when the composition is applied to the skin. The concentrations of these in vanishing cream base is generally from 5%-25% by weight C₁₂-C₂₀ fatty acids and 0.1%-10% by weight fatty acid soap.

Vehicles other than water can include liquid or solid emollients, solvents, humectants, thickeners and powders. Examples of each of these types of vehicles can be used singly or as mixtures of one or more vehicles.

The compositions of the present invention may comprise a wide range of other optional components like antioxidants, binders, biological additives, buffering agents, colorants, thickeners, polymers, astringents, fragrance, humectants, opacifying agents, conditioners, exfoliating agents, pH adjusters, preservatives, natural extracts, essential oils, skin sensates, skin soothing agents, and skin healing agents.

Throughout this specification and claims, by the term "cosmetic composition" is meant a personal care composition. Such compositions may be "leave ons", where the product is left to deliver actives/provide benefits on human substrate e.g. skin (including surfaces on face, hands, body, hair, lips, under arms). Such personal care compositions also include "wash-off" products for cleaning surfaces of human body.

### Antimicrobial for deodorant Application

The present invention provides a deodorant composition for improved antimicrobial actions on skin. The particulate antimicrobial is preferably delivered to the skin through a deodorant composition for protection against malodour. This deodorant composition is made by usual way of making any deodorant formulation.

Deodorant compositions may be deliver through different product format e.g. deo-sprays, deo-sticks or roll-ons.

The particulate antimicrobial is preferably incorporated in 0.05% to 10% by weight of the deo composition, more preferably from 0.1% to 10%, most preferably from 0.2 % to 5 % by weight of the composition.

The deo formulations may further comprise conventional ingredients.

In deo sprays formulation typically 70-99%, preferably 80-95%, most preferably 85-90% of the composition is propellant, solvent and fragrance.

Deo cream compositions of the present invention for use as a deo-stick or roll-ons will include a deodorant active. Most preferable is an astringent salt which combines the properties of deodorancy and antiperspirancy. Amounts of the deodorant active may range from 0.1 to 70%.

Deodorant actives according to the present invention also include materials other than those functioning as antiperspirants. Deodorants should be capable of killing or hindering the growth of microorganisms that generate malodour or that promote the decomposition of body oils into odiferous fatty acids. Amounts of particulate antimicrobial of the invention may range from 0.1 to 1%, preferably 0.2 to 0.5% by weight.

Another component of deodorant cosmetic creams of the present invention is that of a volatile emollient. The emollient is preferably selected from volatile polyorgansiloxanes, C7-C10 hydrocarbons and combinations thereof. These materials may be present in amounts from 1 to 70%, preferably from 10 to 50%, optimally from 25 to 35% by weight. The term "volatile" refers to those materials having a measurable pressure at ambient conditions.

Deodorant cosmetic cream compositions of the present invention will also contains powdered filler/drying agent like starches, talc, fumed silica, finely divided silica, sodium bicarbonate, magnesium aluminium silicate and mixtures thereof. Clays could also be used as a powdered. Amounts of the powdered filler/drying agent will range from 1 to 40%, preferably from 10 to 35%, optimally from 15 to 30% by weight.

Optionally included within the deodorant cosmetic creams of the present invention is that of a non-volatile liquid emollient. Non-volatile polyorganosiloxanes, C₁₂-C₄₀ hydrocarbons and combinations thereof may be suitable for this purpose. Amounts of this material may range from 1 to 40%, preferably from 5 to 25%, optimally from 10 to 20% by weight.

### Antimicrobial particles in hand-wash composition

According to another aspect of the present invention provides a hand-wash composition. The antimicrobial particle is preferably delivered to the hands by a hand-wash composition. This hand-wash composition is made by usual way of making any hand-wash composition.

The particulate antimicrobial is preferably incorporated in 0.01% to 10% by weight of the hand-wash composition, more preferably from 0.1% to 5% by weight of the composition.

The hand-wash composition may further comprise of one or more anionic surfactants, amphoteric and/or zwitterionic surfactants, optional non-ionic surfactants, antimicrobial of the invention, humectants and optionally other minors.

The anionic surfactant may be selected from aliphatic sulphonate, alkyl sulphate, alkyl sulphosuccinates, alkoxylated citrate sulphosuccinates, carboxylates and many others. Zwitterionic surfactants are exemplified by those which can be broadly described as derivatives of aliphatic quaternary ammonium, phosphonium, and sulphonium compounds, in which the aliphatic radicals can be straight or branched chain, and wherein one of the aliphatic substituents contains from about 8 to about 18 carbon atoms and one contains an anionic group, e.g., carboxy, sulphonate, sulphate, phosphate, or phosphonate.

The non-ionic which may be used includes in particular the reaction products of compounds having a hydrophobic group and a reactive hydrogen atom, for example aliphatic alcohols, acids, amides or alkyl phenols with alkylene oxides, especially ethylene oxide either alone or with propylene oxide.

The composition may also comprises of humectants like low molecular weight alcohols such as ethanol, butanol or low molecular weight PEGs or glycerine.

In addition to above mentioned components, other ingredients such as viscosity modifier, pearlizers, perfumes, vitamins, preservatives, dyes etc. may also be included in the composition in minor quantity.

### Examples

### Example 1: Preparation of antimicrobial particle of the invention

5g of Kaolinite (Super shine 90, EICL) was taken in a 500ml of 0.1N HCl (Merck) solution and sonicated for 30 minutes. The pH is then increased to 9 by addition of NaOH (Merck) solution drop wise. To this 10g CPC was added and the suspension was stirred over a magnetic stirrer (Spinpot) for 6 hours while maintaining the temperature of the solution at 75-80 °C. The suspension was then washed with water for about 10 times to remove the excess CPC and a final ethanol (Les Alcools De Commerce) wash was given. The clay was then dried in a hot air oven.

### Example 2: Characterization of prepared clay by FTIR spectroscopy

To determine that CPC was attached on the clay after reaction FTIR- spectroscopy method was utilized. The instrument used was Perkin Elmer instruments, Spectrum One FT-IR Spectrometer. Powder (diffuse reflectance) technique was utilized for this measurement. Clay as control and reacted clay of the invention were grounded with 50% w/w of KBr in a pestle and mortar and then IR was done on these powders. The IR spectrum of the reacted clay was compared against that of pure clay. New peaks were observed in the reacted clay at the wave numbers of 2926 cm⁻¹, 2855 cm⁻¹, 1487 cm⁻¹ and 1466 cm⁻¹. The peaks at 2926 cm⁻¹ and 2855 cm⁻¹ are due to the C-C stretching of the alkyl chain of the CPC, while the peaks at 1487 cm⁻¹ and 1466 cm⁻¹ are due to the ring carbon and nitrogen of the CPC.

### Example 3: Retention comparison of antimicrobial molecule with the particle of the invention onto fabric.

### Deposition, removal and quantification of retention of molecular antimicrobial:

0.5 ml of 180 mM aqueous solution of CPC was dosed using a micro pipette onto three cotton swatches (WFK, 10A, 10X10 cm). The swatches were immediately transferred to a conical flask with 108 ml water (liquid to cloth ratio, L:C = 20). The swatches were rinsed for 2 minutes at 90 rpm using a Haake SW B25 shaker bath at 280C. Then UV-visible spectroscopy was used to determine the concentration of CPC in the rinse water. The instrument used was Cary 50 Probe UV-Visible Spectrophotometer. The UV-visible spectrum of CPC shows peak at 260nm. The intensity of this peak was compared against the calibration curve to determine the concentration of CPC in water. Then previously mentioned steps were repeated twice to estimate molecular antimicrobial loss at each rinse. The summation of three rinses gives the antimicrobial loss after three rinses. The initial value of antimicrobial added was also determined by dosing 0.5 ml of 180 mM CPC and determining its concentration using a standard curve. The concentration thus obtained was multiplied by three to determine the total amount of molecular antimicrobial present initially.

### Deposition, removal and quantification of retention of antimicrobial particle:

0.5 ml of 4g/l of the antimicrobial particle of the invention was dosed onto six cotton swatches (WFK, 10A and 10X10 cm). Three swatches were immediately transferred to a conical flask with 108 ml water (L:C = 20). Then the swatches were rinsed for 2 minutes at 90 rpm using a Haake SW B25 shaker bath at 28°C. Previously mentioned two steps were repeated twice. After that the fabrics were burned and ashed in a muffle furnace (ex. Thermolynl 48000) at 600 °C for 3 hrs. Then the ash was transferred to a small plastic container and weighed (ex Sartorius AG Germany model no CP 2250). Three swatches were ashed without rinsing to determine the initial weight of the antimicrobial particle.

It was found that the average percentage retention (on the fabric) for molecular antimicrobial is 33% whereas the average percentage retention (on the fabric) for antimicrobial particle of the invention is 77%.

### Example 4: Evaluation of antimicrobial activity of the particle of the invention

*Staphylococcus epidermidis* was grown in Tryptone Soya Broth (TSB, HiMedia 30g/L) and shaker incubated for 18hrs at 37°C. The broth was centrifuged. The supernatant was decanted and the pellet was re-suspended 0.9% saline solution. The re-suspended solution was diluted to between 10⁷-10⁸ cfu/mL with saline solution using a optical density calibration curve known to a skill person. 100 micro litres of this suspension of *S.epidermidis* was added to the 96-well Microtitre plate wells. After that the plate was incubated for 2 hrs at 37°C to allow the cells to settle, and removed from the incubator and under sterile conditions to a laminar flow hood and the following assay was performed. 90 micro litres of the supernatant was carefully removed from the wells with a pipette. These cultures were contacted with 100 micro litres of the respective test clay particle suspensions (see Table 1 for final clay particle concentrations). After 1min or 1hour contact time (see Table 1) the 100 micro litres of the clay particle suspensions were removed from each of the wells and discarded. Then the wells were washed with 100 micro litres of sterile distilled water. The plate was then agitated and the water was removed. This was repeated twice (resulting in three washes). 200 micro litres of sterile BHI broth (Brain Heart Infusion broth, 37 g/L, ex Difco) were added to the respective wells in the plate and kept in an IEMS reader to evaluate the optical density at 620nm over 24hrs to monitor whether or not the culture starts to grow as represented in the following Table 1.

**Table 1**

| Concentration of antimicrobial particle | 1 minute contact time | 1 hour contact time |
|---|---|---|
| 4% | No microbial growth after 24 hrs. | No microbial growth after 24 hrs. |
| 1% | No microbial growth after 24 hrs. | No microbial growth after 24 hrs. |
| 0.5% | No microbial growth after 24 hrs. | No microbial growth after 24 hrs. |
| 0.1% | Microbial growth observed after 24 hrs. | No microbial growth after 24 hrs. |
| 0.05 | - | No microbial growth after 24 hrs. |
| 0.01 | - | No microbial growth after 24 hrs. |

From Table 1 it is apparent that the antimicrobial particle works at low concentration for both 1 minute and 1 hour contact time. It also shows that increasing contact time leads to higher antimicrobial activity.

Similarly the above example was repeated with *Streptococcus mutans (S. mutans)* which are gram-positive bacteria. The activity of the particle of the invention was tested against *S. mutans* in solution as well as in the *S.mutans* biofilm.

Solution protocol - A sub culture of *S.mutans* was grown in BHI broth (ex Difco 37 g/L) and grown in a CO₂ incubator having 5% CO₂ at 37°C for 15hrs. The grown culture broth was taken and the concentration was adjusted by dilution with BHI glucose broth (BHI ex Difco 37g/L and Glucose 2%) to 10⁸ cfu/ml. Then 1 mL of the culture was mixed with 9 mL of a saline solution containing various concentrations of particle suspension of the invention resulting to the concentration as indicated in the Table 2 below. The samples were incubated as above for 2hrs. After 2hrs the samples were neutralized in D/E broth (ex Difco 39 g/L) and serially diluted in the same broth according to the usual methods known to a skill person. 1mL of each of the solution was pipetted into respective culture plates and 10 mL of molten BHI agar (ex Difco 52 g/L) was added and mixed and the plates with closed with a lid and left to cool down and solidify for about 30 minutes. The culture plates were transferred to the same incubator and were incubated for 48 hours under above conditions. After incubation the colonies in the plates were counted.

The inhibition data are given below in Table 2.

**Table 2**

| Concentration of particle of invention (in %) | Log reduction of *S. mutans* in solution | Residual *S. mutans* (10⁴ cfu/mL) |
|---|---|---|
| 0 | 0 | 1000 |
| 0.025 | 2.2 | 6.31 |
| 0.05 | 7 | 0 |
| 0.1 | 7 | 0 |
| 0.25 | 7 | 0 |
| 0.5 | 7 | 0 |

Similarly a biofilm test was carried out.

Biofilm protocol - A sub culture of *S.mutans* was grown in a 6 well plate in a broth containing 37 g/L BHI and 2% glucose in an incubator under 5% of CO₂ at 37°C. After 24 hours the media was removed and 2ml of test solutions from the Table 3 below were added to each of the wells and incubated for 2 hours. After that the respective biofilm cells were mixed with the test solution inside the wells of the plate. Then 1 mL of the thus homogenized solution from the wells of the plates were transferred to test tubes containing 9 mL of D/E broth (39 g/L) and mixed. These solutions were serially diluted in the same broth. 1mL of each of the solution was pipetted into respective culture plates and 10 mL of molten BHI agar (ex Difco 52 g/L) was added and mixed and the plates with closed with a lid and left to cool down and solidify for about 30 minutes. The culture plates were transferred to the same incubator and were incubated for 48 hours under above conditions. After incubation the colonies in the plates were counted.

The inhibition data are given below in Table 3.

**Table 3**

| Concentration of particle of invention (in %) | Log reduction of *S. mutans* in solution | Residual *S.mutans* (10⁴ cfu/mL) |
|---|---|---|
| 0 | 0 | 3981.1 |
| 0.1 | 0.5 | 1258.9 |
| 0.2 | 2 | 39.8 |
| 0.5 | 2.6 | 10.0 |
| 1 | 3.2 | 2.5 |

### Example 5: Antimicrobial activity on fabric

Antimicrobial activity of the particle of the invention on laundry was tested.

Cotton swatches having dimension 4 x 4 cm were used in this example. The swatches were soaked in respective compositions as given below for 30 minutes and then washed for 30 minutes in a shaker bath. After that the swatches were rinsed three times with deionized water in a shaker bath. The liquid to cloth ration were maintained at 20 during the washing and rinsing step. The results are given below in Table 4.

**Table 4**

| Composition¹⁾ (g/L in the laundry liquor) | Log reduction |
|---|---|
| Model detergent + CPC (0.02 g/L) | 1.99 |
| Model detergent + Antimicrobial particle of the invention (equivalent amount) | 1.19 |
| *S. epidermidis* (control) | 0 |

| | |
|---|---|
| 1) The model detergent contains: 0.6 g/L Na-LAS; 1.2 g/L Soda; and 0.9 g/L NaCl | |

### Example 6: Comparison of retention between molecular antimicrobial versus antimicrobial particle of the invention.

CPC was taken as the molecular antimicrobial for this purpose and CPC reacted clay particle was taken as the antimicrobial particle of the invention. 1mL of 10gpL CPC solution dosed over hydroxyapatite tile (15mm x 15mm x 5 mm; ex IFGL Bio Ceramics Ltd Kolkata; a common model tooth surface) and kept for 1 min. 19mL of deionized water was added to this and rinsed for 1 min in shaker bath (Orbital Shaking Incubator Model ACM-22065-I) at 180 rpm. The rinsing step was repeated twice with 20 mL of deionized water each. Then UV spectrophotometer (Lambda EZ210 Spectrophotometer) was used to determine the concentration of CPC in the rinse liquors.

It was found that more than 90% of CPC was removed after rinsing.

Similarly 1mL of 5gpL CPC-clay suspension dosed over hydroxyapatite tile and kept for 1min. 19mL of deionized water was added to this and rinsed for 1 min in shaker bath (Orbital Shaking Incubator Model ACM-22065-I) at 180 rpm. The rinsing step was repeated twice with 20 mL of deionized water each. Then Scanning Electron Microscopy (Hitachi S-4700 Scanning Electron Microscope) imaging was done to determine the retention of CPC-clay on the tile.

It was shown that more than 90% of antimicrobial particle of the invention was retained on the tile after rinsing.

## Claims

1. A bipolar antimicrobial particle,
a which precursor is an asymmetric 1:1 or 2:1:1 clay particle comprising alternating tetrahedral and octahedral sheets terminating with a tetrahedral sheet at one external surface plane and an octahedral sheet at another external surface plane,
b with an antimicrobial group attached to the coordinating cation on one of the said external surface plane selected from a quaternary ammonium material comprising a single alkyl or alkenyl long chain having an average chain length greater than or equal to C₂₀, or a quaternary ammonium material selected from Cetylpyridinium chloride (CPC), Cetyltrimethylammonium Chloride (CTAC), Cetyltrimethylammonium Bromide (CTAB), Benzalkonium Chloride (BKC), Benzethonium chloride, cetrimide, Quaternium, polyhexamethylene BH or antimicrobial alcohols selected from phenoxy ethanol, benzyl alcohol, dichlorobenzyl alcohol, dimethyl oxazolidine, DMDM Hydantoin, 2-bromo-2-nitropropane-1,3-diol, diazolidinyl urea, hexachlorophene or antimicrobial phenols selected from triclosan, thymol, dichlorophenol, 2-chloro-4-fluoro phenol, tetrafluorobenzoic acid, cresol, hexylresorcinol, microlides or antimicrobial organic acids/salts selected from Benzoic acid/ sodium benzoate, Salicylic Acid/ Sodium Salicylate, Sorbic Acid/ Potassium Sorbate, sodium hydroxymethyl glycinate, cyclohexane diacetic acid monoamide, chloronicotinic acids, succinic acid, Peracetic acid or Zinc pyrithione, Ketoconazole, Octopirox^{®} or; combinations thereof wherein said antibacterial group is attached to coordinating cations on the external surface of the octahedral surface plane..

2. A particulate antimicrobial as claimed in claim 1 wherein clay: antimicrobial ratio is of between 1:0.001 to 1:0.1

3. A laundry composition comprising an antimicrobial particle as claimed in any one of the preceding claims.

4. A laundry composition as claimed in claim 3 wherein the antimicrobial particle is in the range of 0.01-5% by weight of the composition.

5. A personal wash composition comprising;
a particulate antimicrobial as claimed in claim 1 or 2 ; and
b an acceptable base.

6. A composition as claimed in claimed in claim 5 wherein the acceptable base is a cream, lotion, gel or emulsion.

7. A composition according to any one of claims 5 or 6, wherein the composition is a body wash composition

8. A composition according to any one of claims 5 or 6, wherein the composition is a shampoo composition, preferably anti-dandruff.

9. A deodorant composition comprising ;
a particulate antimicrobial as claimed in claim 1 or 2 ; and
b a deodorant base.

10. A deodorant composition as claimed in claim 9 wherein the deodorant base is a gel or aerosol.

11. A dental hygiene composition comprising an antimicrobial particle as claimed in any one of claims 1 to 2.

12. A process for preparing bipolar antimicrobial particle as claimed in any one of the preceding claims 1 or 2, comprising the steps of:
a contacting the precursor with a mineral acid,
b adjusting the pH of the solution above 8,
c adding a antimicrobial molecule to the mixture,
d heating the mixture to a temperature of 50 - 150 °C for 30 minutes to 10 hours while stirring , and
e Separating the solid product comprising bipolar particulate antimicrobial.

13. A process as claimed in claim 12 wherein the precursor is selected from kaolinite, dickite, halloysite and nacrite, chrysolite, lizardite, and amesite or combinations thereof.

14. Non-therapeutic use of clay particle according to any one of the claims 1 to 2 for increasing antimicrobial activity on fabrics, textiles, human skin and scalp.

## Patentansprüche

1. Bipolares antimikrobielles Teilchen,
a dessen Präkursor ein asymmetrisches 1:1 oder 2:1:1-Tonteilchen ist, umfassend alternierende tetraedrische und oktaedrische Schichten, die mit einer tetraedrischen Schicht an einer äußeren Oberflächenebene und einer oktaedrischen Schicht an einer anderen äußeren Oberflächenebene enden,
b mit einer antimikrobiellen Gruppe, die an das koordinierende Kation auf einer der äußeren Oberflächenschichten gebunden ist, ausgewählt unter einem quaternären Ammoniummaterial, umfassend eine einzelne Alkyl- oder Alkenyl-Langkette mit einer durchschnittlichen Kettenlänge von größer oder gleich C₂₀, oder einem quaternären Ammoniummaterial, ausgewählt unter Cetylpyridiniumchlorid (CPC), Cetyltrimethylammoniumchlorid (CTAC), Cetyltrimethylammoniumbromid (CTAB), Benzalkoniumchlorid (BKC), Benzethoniumchlorid, Cetrimid, Quaternium, Polyhexamethylen (BH), oder antimikrobiellen Alkoholen, ausgewählt unter Phenoxoethanol, Benzylalkohol, Dichlorbenzylalkohol, Dimethyloxazolidin, DMDM Hydantoin, 2-Brom-2-nitropropan-1,3-diol, Diazolidinylharnstoff, Hexachlorophen, oder antimikrobiellen Phenolen, ausgewählt unter Triclosan, Thymol, Dichlorphenol, 2-Chlor-4-fluorphenol, Tetrafluorbenzoesäure, Kresol, Hexylresorcin, Mikroliden, oder antimikrobiellen organischen Säuren/Salzen, ausgewählt unter Benzoesäure/Natriumbenzoat, Salicylsäure Natriumsalicylat, Sorbinsäure/Kaliumsorbat, Natriumhydroxymethylglycinat, Cyclohexandiessigsäuremonoamid, Chlornikotinsäuren, Succinsäure, Peressigsäure oder Zinkpyrithion, Ketoconazol, Octopirox^{®} oder Kombinationen davon, wobei die antimikrobielle Gruppe an koordinierende Kationen auf der äußeren Oberfläche der oktaedrischen Oberflächenebene gebunden ist.

2. Teilchenförmiges antimikrobielles Material nach Anspruch 1, wobei das Verhältnis Ton:antimikrobielles Material zwischen 1:0,001 bis 1:0,1 liegt.

3. Waschmittelzusammensetzung, umfassend ein antimikrobielles Teilchen, wie in einem der vorhergehenden Ansprüche beansprucht.

4. Waschmittelzusammensetzung nach Anspruch 3, wobei das antimikrobielle Teilchen in dem Bereich von 0,01-5 Gewichts-% der Zusammensetzung liegt.

5. Personenwaschzusammensetzung, umfassend
a teilchenförmiges antimikrobielles Material nach Anspruch 1 oder 2 und
b einen annehmbaren Grundbestandteil.

6. Zusammensetzung wie im Anspruch 5 beansprucht, wobei der annehmbare Grundbestandteil eine Creme, eine Lotion, ein Gel oder eine Emulsion ist.

7. Zusammensetzung nach irgendeinem der Ansprüche 5 oder 6, wobei die Zusammensetzung eine Körperwaschzusammensetzung ist.

8. Zusammensetzung nach irgendeinem der Ansprüche 5 oder 6, wobei die Zusammensetzung eine Shampoozusammensetzung ist, vorzugsweise ein Antischuppenmittel.

9. Deodorantzusammensetzung, umfassend:
a teilchenförmiges antimikrobielles Material wie in Anspruch 1 oder 2 beansprucht und
b einen Deodorantgrundbestandteil.

10. Deodorantzusammensetzung wie im Anspruch 9 beansprucht, wobei der Deodorantgrundbestandteil ein Gel oder ein Aerosol ist.

11. Zahnhygienezusammensetzung, umfassend ein antimikrobielles Teilchen wie in irgendeinem der Ansprüche 1 bis 2 beansprucht.

12. Verfahren zur Herstellung eines bipolaren antimikrobiellen Teilchens, wie in irgendeinem der vorhergehenden Ansprüche 1 oder 2 beansprucht, umfassend die Schritte:
a Inkontaktbringen des Präkursors mit einer Mineralsäure,
b Einstellen des pH der Lösung über 8,
c Zugeben eines antimikrobiellen Moleküls zu der Mischung,
d Erhitzen der Mischung auf eine Temperatur von 50-150°C für 30 Minuten bis 10 Stunden unter Rühren und
e Abtrennen des festen Produktes, das das bipolare teilchenförmige antimikrobielle Material umfasst.

13. Verfahren wie in Anspruch 12 beansprucht, wobei der Präkursor unter Kaolinit, Dickit, Halloysit und Nakrit, Chrysolith, Lizardit und Amesit oder Kombinationen davon ausgewählt ist.

14. Nicht-therapeutische Verwendung des Tonteilchens nach irgendeinem der Ansprüche 1 bis 2 zur Steigerung der antimikrobiellen Aktivität auf Geweben, Textilien, menschlicher Haut und der Kopfhaut.

## Revendications

1. Particule antimicrobienne bipolaire,
a. dont le précurseur est une particule d'argile asymétrique 1/1 ou 2/1/1 comprenant des feuillets tétraédriques et octaédriques alternés avec un feuillet tétraédrique au niveau d'un plan de surface externe et un feuillet octaédrique au niveau d'un autre plan de surface externe,
b. avec un groupe antimicrobien rattaché au cation de coordination sur l'un desdits plans de surface externe, choisi parmi un matériau ammonium quaternaire comprenant une seule longue chaîne alkyle ou alcényle ayant une longueur de chaîne moyenne supérieure ou égale à C₂₀, et un matériau ammonium quaternaire choisi parmi le chlorure de cétylpyridinium (CPC), le chlorure de cétyltriméthylammonium (CTAC), le bromure de cétyltriméthylammonium (CTAB), le chlorure de benzalkonium (BKC), le chlorure de benzéthonium, le cétrimide, le quaternium, le polyhexaméthylène BH et les alcools antimicrobiens choisis parmi le phénoxyéthanol, l'alcool benzylique, l'alcool dichlorobenzylique, la diméthyloxazolidine, la DMDM-hydantoïne, le 2-bromo-2-nitropropane-1,3-diol, la diazolidinylurée, l'hexachlorophène et les phénols antimicrobiens choisis parmi le triclosan, le thymol, le dichlorophénol, le 2-chloro-4-fluorophénol, l'acide tétrafluorobenzoïque, le crésol, l'hexylrésorcinol, les microlides et les acides/sels organiques antimicrobiens choisis parmi l'acide benzoïque/le benzoate de sodium, l'acide salicylique/le salicylate de sodium, l'acide sorbique/le sorbate de potassium, l'hydroxyméthylglycinate de sodium, le monoamide d'acide cyclohexanediacétique, les acides chloronicotiniques, l'acide succinique, l'acide peracétique et la zinc-pyrithione, le kétoconazole, l'Octopirox^{®} ; et leurs combinaisons, dans laquelle ledit groupe antibactérien est rattaché à des cations de coordination sur la surface externe du plan de surface octaédrique.

2. Antimicrobien particulaire selon la revendication 1, dans lequel le rapport argile/ antimicrobien est compris entre 1/0,001 et 1/0,1.

3. Composition pour le linge comprenant une particule antimicrobienne selon l'une quelconque des revendications précédentes.

4. Composition pour le linge selon la revendication 3, dans laquelle la particule antimicrobienne représente de 0,01 à 5 % en poids de la composition.

5. Composition de lavage personnel comprenant :
a. un antimicrobien particulaire selon la revendication 1 ou 2 ; et
b. une base acceptable.

6. Composition selon la revendication 5, dans laquelle la base acceptable est une crème, une lotion, un gel ou une émulsion.

7. Composition selon l'une quelconque des revendications 5 et 6, laquelle composition est une composition de lavage pour le corps.

8. Composition selon l'une quelconque des revendications 5 et 6, laquelle composition est une composition de shampooing, de préférence antipelliculaire.

9. Composition de déodorant comprenant :
a. un antimicrobien particulaire selon la revendication 1 ou 2 ; et
b. une base de déodorant.

10. Composition de déodorant selon la revendication 9, dans laquelle la base de déodorant est un gel ou un aérosol.

11. Composition d'hygiène dentaire comprenant une particule antimicrobienne selon l'une quelconque des revendications 1 et 2.

12. Procédé pour préparer une particule antimicrobienne bipolaire selon l'une quelconque des revendications 1 et 2, comprenant les étapes de :
a. mise en contact du précurseur avec un acide minéral,
b. ajustement du pH de la solution à une valeur supérieure à 8,
c. addition d'une molécule antimicrobienne au mélange,
d. chauffage du mélange à une température de 50 à 150°C pendant 30 minutes à 10 heures sous agitation, et
e. séparation du produit solide comprenant l'antimicrobien particulaire bipolaire.

13. Procédé selon la revendication 12, dans lequel le précurseur est choisi parmi la kaolinite, la dickite, l'halloysite et la nacrite, la chrysolite, la lizardite et l'amésite, ainsi que leurs combinaisons.

14. Utilisation non thérapeutique d'une particule d'argile selon l'une quelconque des revendications 1 et 2 pour augmenter l'activité antimicrobienne sur des étoffes, des textiles, la peau humaine, et le cuir chevelu.
